# FASCICULE DE BREVET EUROPEEN

(11) **EP 2 358 665 B1**
(45) Date de publication et mention de la délivrance du brevet: **23.04.2014**
(21) Numéro de dépôt: 09768168.8
(22) Date de dépôt: 10.11.2009
(51) Int. Cl.: C07C 249/14, C07C 251/58, C07C 57/15

(54) **PROCEDE DE PREPARATION DE L'HEMIFUMARATE D'EPLIVANSERINE**
VERFAHREN ZUR HERSTELLUNG VON EPLIVANSERIN HEMIFUMARAT
METHOD FOR PREPARING EPLIVANSERIN HEMIFUMARATE

(30) Priorité: 14.11.2008 FR 0806373
(43) Date de publication de la demande: 24.08.2011
(73) Titulaire: SANOFI, 75008 Paris (FR)
(72) Inventeur: GARCIA, Eric, F-75013 Paris (FR); HOFF, Christian, F-75013 Paris (FR)
(74) Mandataire: Le Coupanec, Pascale A.M.P.
(86) Numéro de dépôt international: PCT/FR2009/052160
(87) Numéro de publication internationale: WO 2010/055255

(56) Documents cités:
- EP-A- 0 373 998
- TAN, PINGZHONG ET AL: "Rapid synthesis of [18F]SR46349B, a potent and selective 5-HT2 receptor antagonist" JOURNAL OF LABELLED COMPOUNDS & RADIOPHARMACEUTICALS , 36(8), 719-28 CODEN: JLCRD4; ISSN: 0362-4803, 1995, XP002525418

## Description

La présente invention concerne un nouveau procédé de préparation de l'hémifumarate d'éplivanserine (1-(2-fluorophényl)-3-(4-hydroxyphényl)-prop-2-en-1-one-O-(2-diméthyl-aminoéthyl) oxime) de formule :

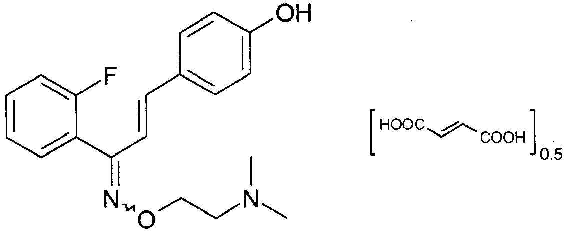

L'éplivanserine peut exister sous la forme de deux isomères, en fonction de la configuration Z ou E de la double liaison C=N, la double liaison C=C étant de configuration E. Au cours de sa synthèse, l'éplivansérine est obtenu sous la forme d'un mélange des deux isomères (Z et E) de la double liaison C=N de la fonction oxime. Ce mélange sera appelé par la suite « éplivanserine base ». L'isomère Z de l'éplivanserine étant plus particulièrement intéressant, il est donc nécessaire de séparer efficacement les deux isomères. Un nouveau procédé de préparation, permettant d'isoler et cristalliser l'isomère désiré de l'éplivanserine, est donc particulièrement attendu.

Les procédés d'isomérisation d'oximes connus de l'état de la technique nécessitent le plus souvent des catalyseurs, du type acides forts ou acides de Lewis (voir par exemple les documents WO98/32758, WO03/033488, ou EP0435687) ou radiations lumineuses (document WO 2006/067092). De façon surprenante, le procédé de préparation selon la présente invention ne nécessite pas de tels additifs.

L'éplivanserine et ses sels, ainsi que leur mode de préparation, sont décrits dans le document EP0373998. Cependant, le procédé décrit ne permet pas de séparer les deux isomères Z et E de façon satisfaisante, c'est-à-dire de façon à obtenir moins de 0,5% de l'isomère E non désiré, tout en évitant une nucléation (formation de nouveaux cristaux) de cet isomère E en fin de réaction. Ce procédé n'est par ailleurs pas transposable à l'échelle industrielle de façon satisfaisante. La présente invention permet de s'affranchir de tels inconvénients, tout en conduisant par ailleurs à une amélioration significative du rendement.

L'hémifumarate d'éplivanserine est notamment décrit dans le document EP0373998 et est connu pour être un antagoniste des récepteurs 5HT_{2A} de la sérotonine (Journal of Pharmacology and Experimental Therapeutics (1992), vol. 262 (2), pp. 759-68). Il est particulièrement utile dans le traitement des troubles du sommeil (Neuropsychopharmacology (1999), vol.21 (3), pp. 455-466 et document WO2007/020337).

Le procédé de synthèse selon la présente invention est caractérisé en ce que l'éplivanserine base est isomérisée et cristallisée, par action de l'acide fumarique, en présence d'un solvant polaire dont la température d'ébullition est supérieure à 100°C, ou d'un mélange de solvants polaires dont la température d'ébullition est supérieure à 100°C, par les étapes suivantes :
i) chauffage de l'éplivanserine base en présence d'acide fumarique, puis refroidissement,
ii) ensemencement,
iii) abaissement par paliers de la température,
iv) filtration et lavage.

Par rapport au document EP0373998, ce nouveau procédé permet d'isoler efficacement l'isomère Z de l'isomère E, tout en évitant la reformation par nucléation de ce dernier, et apporte également une réduction des étapes d'isolement du produit fini, et par là-même un gain de temps significatif. Un doublement du rendement théorique (100 % au lieu de 50 %), est également observé, le procédé étant compatible avec une production industrielle à large échelle.

L'éplivanserine base est isomérisée et cristallisée en présence d'un acide tel que l'acide fumarique, et dans un solvant polaire dont la température d'ébullition est supérieure à 100°C, tel que l'isobutanol (2-butanol), le n-butanol ou le n-pentanol, ou dans un mélange de solvants polaires dont la température d'ébullition est supérieure à 100°C. Comme exemple d'un tel mélange, on peut notamment citer un mélange isobutanol-DMF (diméthylformamide). Selon un mode de réalisation, le solvant est l'isobutanol.

Une température d'ébullition d'un solvant polaire (ou d'un mélange de solvants polaires) supérieure à 100°C permet l'isomérisation en cours de cristallisation et ainsi de développer les conditions optimales nécessaires à la cinétique du procédé.

Le mélange est chauffé de préférence au-delà de 100°C, afin de pouvoir démarrer la cristallisation à l'équilibre d'isomérisation, puis refroidi par paliers jusqu'à la température de filtration. Par exemple, lorsque le solvant est l'isobutanol, le mélange est chauffé à 105°C, ce qui assure d'atteindre l'équilibre d'isomérisation, puis refroidi jusqu'à 100°C.

Par ensemencement, on entend : l'introduction de cristaux de la forme désirée dans le milieu de cristallisation contenant l'éplivanserine base. De préférence, l'ensemencement s'effectuera à l'aide de l'hémifumarate de (1Z, 2E)-1-(2-fluorophenyl)-3-(4-hydroxyphenyl)-prop-2-en-1-one-O-(2-dimethylaminoethyl) oxime, préparé par exemple selon le document EP0373998.

Par solvant polaire, on entend un solvant qui possède un fort moment dipolaire, tel que l'eau, l'acétone, l'ammoniac, les alcools, le DMSO ou le DMF. Parmi les solvants polaires, on distingue deux classes: les solvants protiques, qui peuvent donner lieu à des liaisons hydrogène (comme l'eau, les alcools) et les solvants aprotiques qui ne peuvent pas en former en l'absence de proton labile (comme le DMF ou le DMSO).

Par paliers de température, on entend un abaissement progressif de la température dans le temps, caractérisé par un couple (T, t) : T représentant la température du palier, et t la durée du palier. Entre deux paliers, la température est abaissée de préférence de 5°C par heure, la température finale ne devant pas être inférieure à 70 ± 5 °C. La durée t d'un palier est de préférence comprise entre 30 min et 6 heures, notamment comprise entre 4 heures et 6 heures.

La filtration et le lavage peuvent être réalisés par toute méthode connue de l'homme du métier. Un produit obtenu selon le procédé ci-dessus et particulièrement préféré est l'hémifumarate de 1-(2-fluorophényl)-3-(4-hydroxyphényl)-prop-2-en-1-one-O-(2-diméthylaminoéthyl)oxime comprenant un pourcentage supérieur à 99,5% d'isomère (Z, E).

Un produit obtenu selon le procédé ci-dessus et particulièrement préféré est l'hémifumarate de 1-(2-fluorophenyl)-3-(4-hydroxyphenyl)-prop-2-en-1-one-O-(2-dimethylaminoethyl)oxime comprenant un pourcentage inférieur à 0,5% d'isomère (E, E).

Le sel d'eplivanserine obtenu peut être ensuite purifié par exemple par une étape de cristallisation ou par toute autre méthode connue de l'homme du métier.

L'eplivanserine base peut être obtenue par les étapes suivantes : 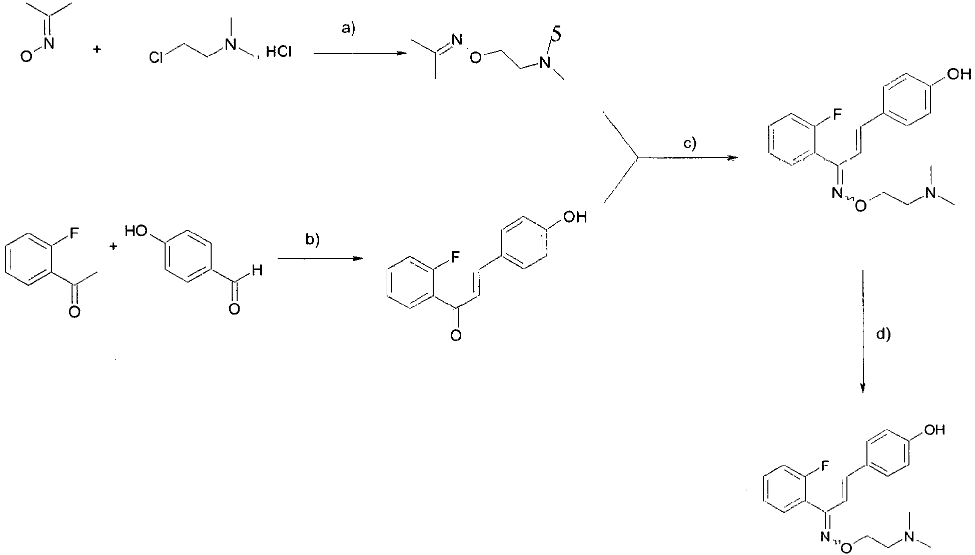
a) l'acétone oxime est condensé avec le dimethylamino-2-chloroéthyle en présence d'une base et d'un solvant, pour former le dimethylaminoacétoxime ;
b) le 4-hydroxybenzaldéhyde est condensée avec la 2-fluoroacétophénone, dans un solvant, pour former la 2-fluorohydroxychalcone;
c) la 2-fluorohydroxychalcone obtenue à l'étape b) est condensée avec le diméthylaminoacétoxime obtenu à l'étape a) dans un solvant pour former l'éplivanserine base;
d) l'éplivanserine base est cristallisée dans un solvant.

Le solvant utilisé à l'étape d) est le toluène.

L'éplivanserine base obtenue à l'étape c) est obtenue à partir de la 2-fluorohydroxychalcone et du diméthylaminoacétoxime, en présence d'un solvant tel que le n-butanol.

La 2-fluorohydroxychalcone est obtenue à partir du 4-hydroxybenzaldéhyde et de la 2-fluoroacétophénone, dans un solvant choisi parmi : l'éthanol chlorhydrique, le pentanol chlorhydrique, l'isopropanol chlorhydrique, le n-butanol chlorhydrique, à une température inférieure à 30°C. Selon un mode de réalisation, le solvant est l'éthanol chlorhydrique.

Le dimethylaminoacétoxime est obtenu à partir de l'acétone oxime et du dimethylamino-2-chloro-éthyle en présence d'une base telle que NaOH ou KOH, et d'un solvant polaire, tel que le THF (tétrahydrofurane). Selon un mode de réalisation, la base est KOH. Le diméthylacétoxime peut être isolé sous la forme d'un sel d'oxalate ou conservé sous forme de base, en solution dans un mélange THF/MTBE (méthyle *tert*-butyle éther).

### Exemple 1 : Synthèse de l'hémifumarate d'éplivansérine

80 kg de 2-fluoroacétophénone et 67 Kg de 4-hydroxybenzaldéhyde sont dissous dans l'éthanol. Une solution d'éthanol chlorhydrique (au moins 64 Kg) est additionnée sur le mélange puis le milieu est agité à une température inférieure à 30°C pendant 5h15. Le milieu est refroidi, on observe la cristallisation de la 2-fluorohydroxychalcone qui est filtrée et lavée par du toluène, puis séchée sous vide à une température inférieure à 60°C.

Rendement chimique à partir de la 2-fluoroacétophénone : 75±15%.

Une solution aqueuse de dimethylamino-2-chloroéthyle (DMAC) (246 Kg d'une solution à 65% m/m) est additionnée à un mélange d'acétone oxime (162 Kg), de potasse (160 Kg) dans le THF (600L). Le milieu réactionnel est porté au reflux pendant au moins 5h. Le milieu est ensuite dilué avec de l'eau, une solution aqueuse de NaCl et une solution aqueuse de soude puis extrait au MTBE. La phase organique est lavée par une solution de NaCl et de soude pour conduire au diméthylaminoacétoxime (DMA acétoxime) en solution MTBE, utilisée telle quelle dans l'étape de couplage avec la 2-fluorohydroxychalcone.

La solution de diméthylaminoacétoxime telle que préparée ci-dessus (105 Kg de diméthylaminoacétoxime, 100% base) est ajoutée à une solution d'acide oxalique (100 Kg) dans le 1-butanol (600L). Le THF et le MTBE sont strippés par du 1-butanol et le sel d'oxalate obtenu est mis en réaction avec la 2-fluorohydroxychalcone (90 Kg) en présence d'HCl aqueux (202 Kg). Le mélange est porté à 102°C pendant au moins 6 heures en distillant l'acétone au fur et à mesure de sa formation et en la remplaçant par du 1-butanol. En fin de réaction, le 1-butanol est remplacé par de l'eau par distillation. La phase aqueuse obtenue est lavée au MTBE, ramenée à pH neutre par addition d'hydroxyde d'ammonium concentré puis porté à pH 9-9,5 par addition d'une solution de carbonate de sodium. Le solide obtenu est alors filtré, lavé à l'eau et séché. Le mélange éplivanserine base brut est ensuite dissous dans du toluène (1580L) à environ 95°C. La solution est filtrée à chaud sur un media filtrant imprégné de charbon actif puis la solution est refroidie, permettant la cristallisation de l'éplivanserine base qui est filtré et séché sous vide à une température inférieure à 70°C.

Rendement chimique à partir de la 2-fluorohydroxychalcone : 80±15%

Le mélange d'isomères Z et E (eplivanserine base) (107 Kg) et l'acide fumarique (19 Kg) sont dissous dans l'isobutanol (environ 1700L) et le mélange est porté à reflux jusqu'à dissolution totale. Le milieu est maintenu à reflux pendant environ une demi-heure. Le milieu est ensuite refroidi à 100°C avec une rampe de refroidissement d'environ -10°C/h. Environ 2% d'hémifumarate de (1Z,2E)-1-(2-fluorophenyl)-3-(4-hydroxyphenyl)-prop-2-en-1-one-O-(2-dimethylaminoethyl) oxime est introduit dans la solution. Une isotherme d'une ½ heure est respectée avant de refroidir la suspension à 90°C environ avec une rampe de refroidissement de -5°C/h. Un palier de 6h à 90°C est respecté avant de refroidir à nouveau la suspension à 85°C avec une rampe de refroidissement de -5°C/h. Un palier de 6h à 85°C est respecté avant de refroidir à 82,5°C avec une rampe de refroidissement de -5°C/h. Un palier de 4h à la température de 82,5°C est maintenu avant de refroidir à 80°C avec une rampe de refroidissement de -5°C/h. La température est maintenue à 80°C pendant 4 heures, ensuite refroidie à 70°C avec une rampe de refroidissement de -2,5°C/h. La température est maintenue en isotherme à 70°C environ une demi-heure avant de filtrer la suspension. La poudre obtenue est lavée avec environ 170 L d'éthanol puis séchée à environ 45°C sous vide. On obtient ainsi environ 96Kg de poudre d'hémifumarate de (1Z,2E)-1-(2-fluorophenyl)-3-(4-hydroxyphenyl)-prop-2-en-1-one-O-(2-dimethylaminoethyl) oxime contenant moins de 0,5% de l'isomère (E,E), soit un rendement d'environ 77%.

De manière comparable, on peut obtenir des résultats similaires en utilisant comme solvant polaire par exemple le n-butanol ou le n-pentanol, ou comme mélange de solvants polaires, un mélange (95/5) isobutanol-DMF.

## Revendications

1. Procédé de préparation de l'hémifumarate d'éplivanserine, **caractérisé en ce que** l'épilvanserine base est Isomérisée et cristallisée, par action de l'acide fumarique, en présence d'un solvant polaire dont la température d'ébullition est supérieure à 100°C ou d'un mélange de solvants polaires dont la température d'ébullition est supérieure à 100°C, par les étapes suivantes :
i) chauffage de l'éplivanserine base en présence d'acide fumarique, puis refroidissement,
ii) ensemencement,
iii) abaissement par paliers de la température,
iv) filtration et lavage.

2. Procédé selon la revendication 1, dans lequel le solvant polaire est l'isobutanol.

3. Procédé selon la revendication 1, dans lequel le solvant polaire est le n-butanol.

4. Procédé selon la revendication 1, dans lequel le solvant polaire est le n-pentanol.

5. Procédé selon la revendication 1, dans lequel le mélange de solvants polaires est un mélange Isobutanol-DMF.

6. Procédé selon la revendication 1, dans lequel le produit obtenu est l'hémifumarate de (1Z, 2E)-1-(2-fluorophenyl)-3-(4-hydroxyphenyl)-prop-2-en-1-one-O-(2-dimethylaminoethyl) oxime.

7. Procédé selon la revendication 1 ou 2, **caractérisé en ce qu'**au point i), le mélange est chauffé à 105 °C puis refroidi à 100°C.

8. Procédé selon la revendication 1, **caractérisé en ce qu'**au point ii), l'ensemencement est réalisé avec l'hémifumarate® de (1Z, 2E)-1-(2-fluorophenyl)-3-(4-hydroxyphenyl)-prop-2-en-1-one-O-(2-dimethylaminoethyl)oxime.

9. Procédé selon la revendication 1, **caractérisé en ce qu'**au point iii), la température entre deux paliers est abaissée de 5°C par heure, jusqu'à 70°C.

10. Procédé selon la revendication 1, **caractérisé en ce qu'**au point iii), la durée d'un palier est comprise entre 30 minutes et 6 heures.

11. Procédé selon la revendication 1 ou 7, **caractérisé en ce qu'**au point iii), la durée d'un palier est comprise entre 4 heures et 6 heures.

12. Procédé selon la revendication 1, **caractérisé en ce que** l'éplivanserine base est obtenue par les étapes suivantes : 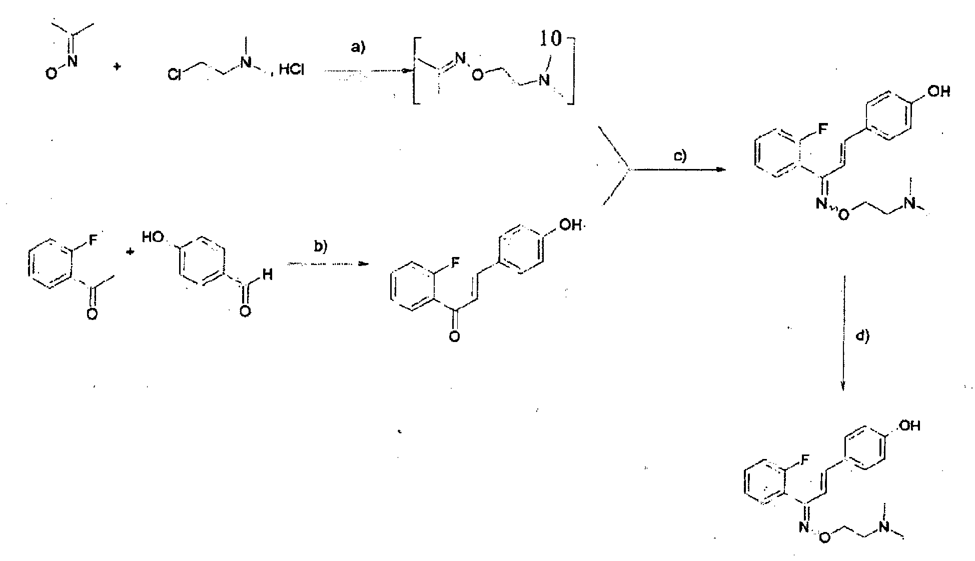
a) l'acétone oxime est condensé avec le diméthylamino-2-chloroéthyle en présence d'une base et d'un solvant, pour former le diméthylaminoacétoxime ;
b) le 4-hydroxybenzaldéhyde est condensée avec la 2-fluoroacétophénone, dans un solvant, pour former la 2-fluorohydroxychalcone;
c) la 2-fluorohydroxychalcone obtenue à l'étape b) est condensée avec le diméthylaminoacétoxime obtenu à l'étape a) dans un solvant, pour former l'éplivanserine base;
d) l'éplivanserine base est cristallisée dans un solvant.

13. Procédé de synthèse selon la revendication 9, **caractérisé en ce que** dans l'étape a), la base est KOH et le solvant polaire est le THF.

14. Procédé de synthèse selon la revendication 9, **caractérisé en ce que** dans l'étape b), le solvant est l'éthanol chlorhydrique.

15. Procédé de synthèse selon la revendication 9 **caractérisé en ce que** dans l'étape c), le solvant est le n-butanol.

16. Procédé de synthèse selon la revendication 9 **caractérisé en ce que** dans l'étape d), le solvant est le toluène.

## Patentansprüche

1. Verfahren zur Herstellung von Eplivanserinhemifumarat, **dadurch gekennzeichnet, dass** die Eplivanserin-Base durch Einwirken von Fumarsäure in Gegenwart eines polaren Lösungsmittels, dessen Siedepunkt über 100°C liegt, oder einer Mischung von polaren Lösungsmitteln, deren Siedepunkt über 100°C liegt, durch die folgenden Schritte:
i) Erhitzen der Eplivanserin-Base in Gegenwart von Fumarsäure und anschließendes Abkühlen,
ii) Animpfen,
iii) stufenweises Erniedrigen der Temperatur,
iv) Filtrieren und Waschen
isomierisiert und kristallisiert wird.

2. Verfahren nach Anspruch 1, wobei es sich bei dem polaren Lösungsmittel um Isobutanol handelt.

3. Verfahren nach Anspruch 1, wobei es sich bei dem polaren Lösungsmittel um n-Butanol handelt.

4. Verfahren nach Anspruch 1, wobei es sich bei dem polaren Lösungsmittel um n-Pentanol handelt.

5. Verfahren nach Anspruch 1, wobei es sich bei der Mischung von polaren Lösungsmitteln um eine Isobutanol-DMF-Mischung handelt.

6. Verfahren nach Anspruch 1, wobei es sich bei dem erhaltenen Produkt um (1Z,2E)-1-(2-Fluorphenyl)-3-(4-hydroxyphenyl)prop-2-en-1-on-O-(2-dimethylaminoethyl)oxim-Hemifumarat handelt.

7. Verfahren nach Anspruch 1 oder 2, **dadurch gekennzeichnet, dass** in Punkt i) die Mischung auf 105°C erhitzt und anschließend auf 100°C abgekühlt wird.

8. Verfahren nach Anspruch 1, **dadurch gekennzeichnet, dass** in Punkt ii) das Animpfen mit (1Z,2E)-1-(2-Fluorphenyl)-3-(4-hydroxyphenyl)prop-2-en-1-on-O-(2-dimethylaminoethyl)oxim-Hemifumarat erfolgt.

9. Verfahren nach Anspruch 1, **dadurch gekennzeichnet, dass** in Punkt iii) die Temperatur zwischen zwei Stufen um 5°C pro Stunde bis auf 70°C erniedrigt wird.

10. Verfahren nach Anspruch 1, **dadurch gekennzeichnet, dass** in Punkt iii) die Dauer einer Stufe zwischen 30 Minuten und 6 Stunden liegt.

11. Verfahren nach Anspruch 1 oder 7, **dadurch gekennzeichnet, dass** in Punkt iii) die Dauer einer Stufe zwischen 4 Stunden und 6 Stunden liegt.

12. Verfahren nach Anspruch 1, **dadurch gekennzeichnet, dass** die Eplivanserin-Base über die folgenden Schritte erhalten wird: 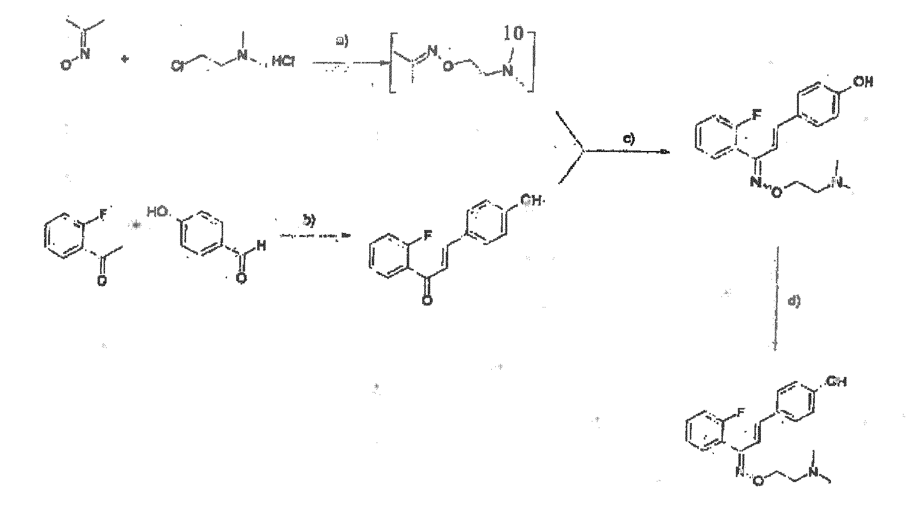
a) das Acetonoxim wird mit dem Dimethylamino-2-chlorethyl in Gegenwart einer Base und eines Lösungsmittels zu Dimethylaminoacetoxim kondensiert;
b) das 4-Hydroxybenzaldehyd wird mit dem 2-Fluoracetophenon in einem Lösungsmittel zu 2-Fluorhydroxychalcon kondensiert;
c) das in Schritt b) erhaltene 2-Fluorhydroxychalcon wird mit dem in Schritt a) erhaltenen Dimethylaminoacetoxim in einem Lösungsmittel zur Eplivanserin-Base kondensiert,
d) die Eplivanserin-Base wird in einem Lösungsmittel kristallisiert.

13. Syntheseverfahren nach Anspruch 9, **dadurch gekennzeichnet, dass** es sich in Schritt a) bei der Base um KOH und bei dem polaren Lösungsmittel um THF handelt.

14. Syntheseverfahren nach Anspruch 9, **dadurch gekennzeichnet, dass** es sich in Schritt b) bei dem Lösungsmittel um Salzsäure in Ethanol handelt.

15. Syntheseverfahren nach Anspruch 9, **dadurch gekennzeichnet, dass** es sich in Schritt c) bei dem Lösungsmittel um n-Butanol handelt.

16. Syntheseverfahren nach Anspruch 9, **dadurch gekennzeichnet, dass** es sich in Schritt d) bei dem Lösungsmittel um Toluol handelt.

## Claims

1. Process for the preparation of eplivanserin hemifumarate, **characterized in that** the eplivanserin base is isomerized and crystallized, by the action of fumaric acid, in the presence of a polar solvent, the boiling point of which is greater than 100°C, or of a mixture of polar solvents, the boiling point of which is greater than 100°C, by the following stages:
i) heating the eplivanserin base in the presence of fumaric acid and then cooling,
ii) seeding,
iii) lowering the temperature via stationary phases,
iv) filtering and washing.

2. Process according to Claim 1, in which the polar solvent is isobutanol.

3. Process according to Claim 1, in which the polar solvent is n-butanol.

4. Process according to Claim 1, in which the polar solvent is n-pentanol.

5. Process according to Claim 1, in which the mixture of polar solvents is an isobutanol/DMF mixture.

6. Process according to Claim 1, in which the product obtained is (1Z, 2E)-1-(2-fluorophenyl)-3-(4-hydroxyphenyl)prop-2-en-1-one-O-[2-(dimethylamino) ethyl]oxime hemifumarate.

7. Process according to Claim 1 or 2, **characterized in that**, in point i), the mixture is heated to 105°C and then cooled to 100°C.

8. Process according to Claim 1, **characterized in that**, in point ii), seeding is carried out with (1Z, 2E)-1-(2-fluorophenyl)-3-(4-hydroxyphenyl)-prop-2-en-1-one-O-[2-(dimethylamino)ethyl]oxime hemifumarate.

9. Process according to Claim 1, **characterized in that**, in point iii), the temperature between two stationary phases is lowered by 5°C per hour, down to 70°C.

10. Process according to Claim 1, **characterized in that**, in point iii), the duration of a stationary phase is between 30 minutes and 6 hours.

11. Process according to Claim 1 or 7, **characterized in that**, in point iii), the time of a stationary phase is between 4 hours and 6 hours.

12. Process according to Claim 1, **characterized in that** the eplivanserin base is obtained by the following stages: 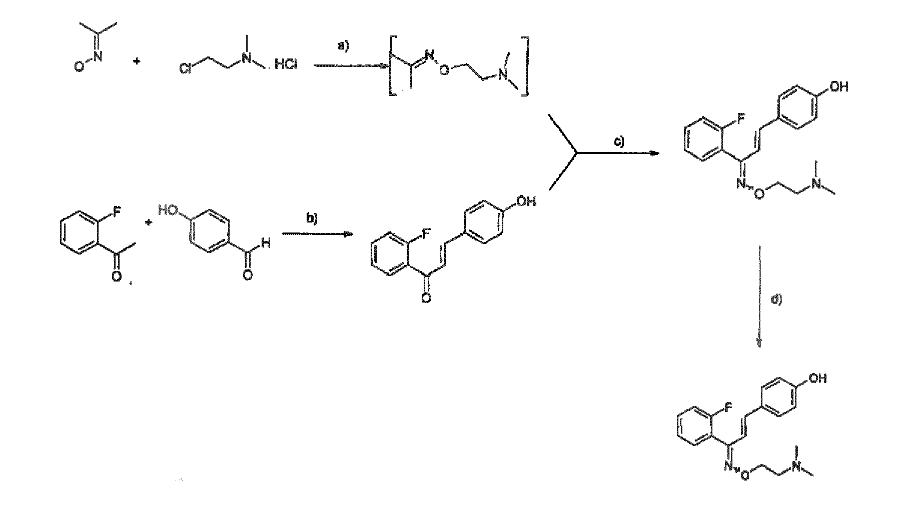
a) acetone oxime is condensed with (2-chloroethyl)dimethylamine in the presence of a base and of a solvent, to form the dimethylaminoacetoxime;
b) 4-hydroxybenzaldehyde is condensed with 2-fluoroacetophenone in a solvent, to form the 2-fluorohydroxychalcone;
c) the 2-fluorohydroxychalcone obtained in stage b) is condensed with the dimethylaminoacetoxime obtained in stage a) in a solvent, to form the eplivanserin base;
d) the eplivanserin base is crystallized in a solvent.

13. Synthetic process according to Claim 9, **characterized in that**, in stage a), the base is KOH and the polar solvent is THF.

14. Synthetic process according to Claim 9, **characterized in that**, in stage b), the solvent is ethanolic hydrochloric acid solution.

15. Synthetic process according to Claim 9, **characterized in that**, in stage c), the solvent is n-butanol.

16. Synthetic process according to Claim 9, **characterized in that**, in stage d), the solvent is toluene.
